# EUROPÄISCHE PATENTSCHRIFT

(11) **EP 0 818 974 B1**
(45) Veröffentlichungstag und Bekanntmachung des Hinweises auf die Patenterteilung: **04.07.2001**
(21) Anmeldenummer: 96905782.7
(22) Anmeldetag: 23.02.1996
(51) Int. Cl.: A61B 17/32

(54) **SCHERENFÖRMIGES, CHIRURGISCHES WERKZEUG UND VERFAHREN ZU DESSEN HERSTELLUNG**
SCISSORS-LIKE TOOL FOR A SURGICAL INSTRUMENT AND PROCESS FOR PRODUCING IT
OUTIL CHIRURGICAL EN FORME DE CISEAUX ET SON PROCEDE DE FABRICATION

(30) Priorität: 04.04.1995 DE 19512559
(43) Veröffentlichungstag der Anmeldung: 21.01.1998
(73) Patentinhaber: Aesculap AG & Co. KG, 78532 Tuttlingen (DE)
(72) Erfinder: GIORDANO, Nicola, D-78056 Villingen-Schwenningen (DE); LUTZE, Theodor, D-78582 Balgheim (DE); WEISSHAUPT, Dieter, D-78194 Immendingen (DE); WIENEKE, Paul, D-78604 Reitheim-Weilheim (DE)
(74) Vertreter: Hoeger, Stellrecht & Partner
(86) Internationale Anmeldenummer: EP9600750
(87) Internationale Veröffentlichungsnummer: WO9631163

(56) Entgegenhaltungen:
- EP-A- 0 003 668
- WO-A-87/05483
- WO-A-91/02493
- US-A- 5 241 968
- US-A- 5 352 235

## Beschreibung

Die Erfindung betrifft ein scherenförmiges Werkzeug für ein chirurgisches Instrument mit zwei miteinander verbundenen Armen, die durch mehr oder weniger weites außenseitiges Übergreifen einer relativ zu dem scherenförmigen Werkzeug axial verschiebbaren, dieses umgreifenden Hülse elastisch zwischen einer gespreizten Offenstellung und einer angenäherten Schließstellung verschwenkbar sind.

Weiterhin betrifft die Erfindung ein Verfahren zur Herstellung eines solchen scherenförmigen Werkzeugs.

Ein derartiges scherenförmiges Werkzeug ist beispielsweise aus der W094/08521 bekannt. Um derartige scherenförmige Werkzeuge in endoskopischen Operationen verwenden zu können, ist es notwendig, daß sehr kleine Dimensionen eingehalten werden, beispielsweise ist es wünschenswert, den Schaft eines entsprechenden chirurgischen Instruments, in dem ein solches scherenförmiges Werkzeug verwendet wird, mit einem Außendurchmesser von 2 mm auszubilden.

Es ist dann praktisch unmöglich, übliche scherenförmige Werkzeuge zu verwenden, die zwei mittels eines Schlusses gelenkig miteinander verbundene Teile umfassen.

Bei dem bekannten scherenförmigen Werkzeug der WO94/08521 wird dieses scherenförmige Werkzeug aus zwei getrennten Teilen aufgebaut, die nach Art von Scherenblättern ausgebildet sind und die in einem hinteren Teil durch Verschweißen oder Formschluß dauerhaft miteinander verbunden werden. Die Herstellung ist daher außerordentlich kompliziert, da zwei Einzelteile mit sehr kleinen Abmessungen hergestellt und sorgfältig miteinander verbunden werden müssen. Es ist außerdem in der Praxis fast unmöglich, Scherenblätter der in dieser Veröffentlichung gezeigten Art mit Abmessungen herzustellen, die beispielsweise in der Querrichtung unter 1 mm liegen.

Es ist Aufgabe der Erfindung, ein scherenförmiges Werkzeug der gattungsgemäßen Art so auszubilden, daß die Herstellung auch bei sehr kleinen Außenabmessungen möglich ist.

Diese Aufgabe wird bei einem scherenförmigen Werkzeug der eingangs beschriebenen Art erfindungsgemäß dadurch gelöst, daß zur Ausbildung der Arme ein Stab aus elastischem Material von seinem freien Ende her einen diametralen vorderen Einschnitt aufweist, an den sich ein weiterer diametraler hinterer Einschnitt anschließt, der gegenüber dem vorderen Einschnitt um 90° um die Stablängsachse gedreht ist, daß die beiden Einschnitte durch quer zur Stablängsachse verlaufende Quereinschnitte miteinander verbunden sind, die sich jeweils über gegenüberliegende Quadranten des Querschnitts zwischen den beiden diametralen Einschnitten erstrecken, während die beiden dazwischenliegenden Quadranten keine derartigen Quereinschnitte aufweisen, und daß die beiden Arme in der Ebene des vorderen diametralen Einschnitts dauerhaft in die gespreizte Offenstellung aufgebogen sind.

Mit einem scherenförmigen Werkzeug dieser Art ist es möglich, zur Herstellung von einem einfachen Stab auszugehen und diesen durch diverse Einschnitte und das Aufbiegen der dadurch entstandene Arme zu bearbeiten, man erhält auf diese Weise ein einstückiges scherenförmiges Werkzeug, bei dem keinerlei Verbindungsarbeiten notwendig sind. Es ist daher möglich, auch bei sehr kleinen Abmessungen ein entsprechendes Werkzeug herzustellen, beispielsweise kann der Stab einen Außendurchmesser von 0,7 mm haben.

Günstig ist es außerdem, wenn die beiden Arme zusätzlich in der Ebene des hinteren diametralen Einschnitts dauerhaft einander überlappend gegeneinander gebogen sind. Dadurch liegen die Schneidkanten bei der Schneidbewegung immer elastisch aneinander an und gewährleisten einen sauberen Schnitt.

Der Stab kann bei einem bevorzugten Ausführungsbeispiel einen kreisförmige Querschnitt aufweisen, insbesondere handelt es sich dabei um einen Federdraht.

Bei einer bevorzugten Ausführungsform ist vorgesehen, daß der Stab zwei seitliche diametral gegenüberliegende außenseitige Abflachungen aufweist, die mit dem vorderen diametralen Einschnitt einen spitzen Winkel einschließen und zusammen mit der Innenfläche des vorderen diametralen Einschnitts eine Schneide ausbilden.

Bei einer bevorzugten Ausführungsform der Erfindung ist dabei vorgesehen, daß der Stab aus einer pseudoelastischen Formgedächtnislegierung mit spannungsinduzierter Martensitbildung bei Körper- und Umgebungstemperatur besteht. Derartige Legierungen sind an sich bekannt (EP 145 166 B1), sie zeichnen sich dadurch aus, daß sie in einem bestimmten Temperaturbereich in einem austenitischen Zustand vorliegen, wenn sie unverspannt sind. Wenn sie dagegen verspannt werden, ergibt sich ein Phasenübergang unter Martensitbildung, der reversibel ist. Der Phasenübergang erfolgt isotherm und führt dazu, daß dieses Material außerordentlich hohe elastische Verformungen zuläßt, die größer sind als die elastischen Verformungen innerhalb einer unveränderten Phase. Durch den Phasenübergang wird bei diesem Material eine stärkere, reversible Verformung möglich, man spricht daher von einem pseudoelastischen Effekt.

Ein solches Material kann beispielsweise eine Nickel-Titan-Legierung sein. Diese ist dabei so auszuwählen, daß der beschriebene pseudoelastische Effekt bei Temperaturen zu beobachten ist, die der Körpertemperatur und der Einsatztemperatur des chirurgischen Instruments entspricht, die also beispielsweise zwischen 15° C und 45° C liegt.

Bei einer ganz besonders bevorzugten Ausführungsform ist vorgesehen, daß der Stab durch die Schub- und Zugstange eines Rohrschaftinstruments gebildet wird, die in einem Rohr relativ zu diesem in axialer Richtung verschiebbar ist. Bei dieser Ausgestaltung ist das scherenförmige Werkzeug also einstückig mit der Schub- und Zugstange ausgebildet, die ohnehin bei Rohrschaftinstrumenten zur Übertragung der Öffnungs- und Schließbewegung verwendet wird. Es ist daher nicht mehr notwendig, spezielle Kraftumlenkungsglieder im Werkzeugbereich vorzusehen oder Verbindungen herzustellen, das freie Ende der Schub- und Zugstange wird durch die genannten Einschnitte und Verformungen zu einem scherenförmige Werkzeug umgebildet.

Weiterhin kann vorgesehen sein, daß der Stab anschließend an das scherenförmige Werkzeug einen Biegebereich mit reduziertem Querschnitt aufweist. Dadurch ist es möglich, einen solchen Stab in einem gebogenen Rohr anzuordnen, der Biegebereich mit reduziertem Querschnitt wird dann im gebogenen Bereich des den Stab aufnehmenden Rohrs angeordnet.

Dabei kann vorgesehen sein, daß auf dem Stab und innerhalb des Rohrs als das scherenförmige Werkzeug umgreifende Hülse ein flexibler Schlauch angeordnet ist. Dieser kann unabhängig von der Biegung des Rohrs und des Stabs in axialer Richtung gegenüber dem Stab verschoben werden und kann zum öffnen und Schließen des scherenförmigen Werkzeugs verwendet werden.

Bei einer abgewandelten Ausführungsform ist vorgesehen, daß die Arme in der Ebene des vorderen diametralen Einschnitts nach innen abgekröpft sind. Dadurch ist es möglich, die beiden Arme unter einem vergrößerten Schnittwinkel anzuordnen, dies kann für bestimmte Anwendungsbereiche von Vorteil sein.

Die genannte Aufgabe wird erfindungsgemäß durch ein Verfahren zur Herstellung eines scherenförmigen Werkzeugs gelöst, welches gekennzeichnet ist durch die folgenden Schritte:
a. man bringt durch ein linienförmig trennendes Werkzeug von der Stirnseite eines Stabs aus elastischem Material einen diametralen vorderen Einschnitt in den Stab ein und trennt diesen im Einschnittsbereich dadurch in zwei Hälften,
b. am Ende des vorderen Einschnitts dreht man den Stab relativ zum trennenden Werkzeug um die Stablängsachse um 90° und erzeugt dadurch zwei sich über 90° erstreckende Quereinschnitte,
c. man bringt durch das linienförmig trennende Werkzeug einen diametralen hinteren Einschnitt in den Stab ein, der um 90° gegenüber dem diametralen vorderen Einschnitt gedreht ist und sich von den Quereinschnitten nach hinten in den Stab erstreckt;
d. man biegt die durch die Einschnitte entstandenen Arme in der Ebene des diametralen vorderen Einschnitts dauerhaft in eine gespreizte Offenstellung auf.

Daraus wird deutlich, daß die Herstellung des scherenförmigen Werkzeug ganz einfach durch zwei unterschiedliche Arbeitsschritte zu erreichen ist, nämlich das Einbringen von Einschnitten einerseits und das Aufbiegen der entstandenen Arme andererseits. Das Einbringen der Einschnitte erfolgt durch ein trennendes Werkzeug, wobei das Werkzeug relativ zu dem Stab in Stablängsrichtung verschoben wird. Innerhalb der Verschiebestrecke werden Werkzeug und Stab einmal um 90° um die Stablängsachse relativ zueinander verdreht, ansonsten erfolgt lediglich ein Vorschub des Stabs relativ zu dem trennenden Werkzeug, um die Einschnitte herzustellen. Da dieses Herstellungsverfahren relativ einfach ist und keine komplizierten Verschiebewege benötigt, ist es auch möglich, eine solche Ausgestaltung in einem Stab mit sehr kleinen Abmessungen vorzunehmen.

Um eine gegenseitige Vorspannung der beiden Arme im Bereich der Schnittkante sicherzustellen, ist es weiterhin vorteilhaft, wenn man die beiden Arme zusätzlich in der Ebene des diametralen hinteren Einschnitts dauerhaft aufeinander zu biegt und sie dadurch in eine gegeneinander vorgespannte Position bringt.

Bei einer bevorzugten Ausführungsform der Erfindung ist vorgesehen, daß man zur dauerhaften Fixierung der Verbiegung der Arme in die Offenstellung und in eine gegeneinander vorgespannte Position den Stab einer Wärmebehandlung unterzieht. Diese sich unmittelbar an die genannten Verbiegungen anschließende Wärmebehandlung führt zu eine Gefügeumwandlung, so daß das scherenförmige Werkzeug in der Offenstellung und in der gegeneinander vorgespannten Position sozusagen eine Ruhestellung erfährt, aus der die Arme bei einer Verbiegung elastisch ausgelenkt werden.

Als trennendes Werkzeug kann man insbesondere eine Drahterosionseinrichtung verwenden, es wäre aber auch möglich, andere derartige Werkzeuge einzusetzen, beispielsweise einen Laserstrahl oder einen Elektronenstrahl. Wesentlich ist, daß das trennende Werkzeug linienförmig arbeitet, so daß längs dieser Linie eine Trennung des Materials erfolgt.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines Stabs mit einem scherenförmigen Werkzeug einschließlich der Darstellung eines trennenden Werkzeugs in verschiedenen Positionen;
- Figur 2:: eine perspektivische Seitenansicht des scherenförmigen Werkzeugs einer ersten Ausführungsform in geöffneter Stellung;
- Figur 3:: eine Draufsicht auf das scherenförmige Werkzeug der Figur 1;
- Figur 4:: eine schematische Seitenansicht eines weiteren bevorzugten Ausführungsbeispiels eines scherenförmigen Werkzeugs mit nach innen abgekröpften Armen;
- Figur 5:: eine perspektivische Ansicht des scherenförmigen Werkzeugs der Figur 4 und
- Figur 6:: eine Ansicht eines scherenförmigen Werkzeugs mit aufgebogenen Armen und mit gegeneinander verbogenen Armen bei der dauerhaften Fixierung dieser Verbiegungen.

Zur Herstellung eines scherenförmigen Instruments wird ausgegangen von einem Stab oder Draht 1 aus einem elastischen Material, insbesondere aus einer Metall-Legierung mit pseudoelastischem Verhalten. Dieses Material kann beispielsweise eine Nickel-Titan-Legierung sein. Der Außendurchmesser des einen kreisförmigen Querschnitt aufweisenden Drahts 1 liegt beispielsweise in der Größenordnung von 1 mm, gegebenenfalls kann er sogar noch geringer sein.

Dieser Draht 1 bildet gleichzeitig die Schub- und Zugstange eines in der Zeichnung nicht dargestellten chirurgischen Rohrschaftinstruments, diese Schub- und Zugstange wird im Inneren eines starren Rohrs dieses Instruments angeordnet und ist gegenüber diesem starren Rohr in Längsrichtung verschiebbar, wie dies beispielsweise aus der bereits erwähnte WO94/08521 bekannt ist.

Das vordere Ende dieses Drahts 1 wird in der nachstehend beschriebenen Art und Weise zu einem scherenförmigen Werkzeug 2 ausgebildet, das dann am vorderen Ende des Rohrschaftinstruments positioniert wird und von der gegenüberliegenden Seite des Rohrschaftinstruments geöffnet und geschlossen werden kann.

Zunächst wird der Draht 1 an seinem Umfang mit zwei diametral gegenüberliegenden Abflachungen 3 versehen, die sich von der Stirnseite 4 über eine bestimmte Länge erstrecken, beispielsweise bei einem Draht 1 mit einem Durchmesser von 0,7 mm über eine Länge von 5 mm.

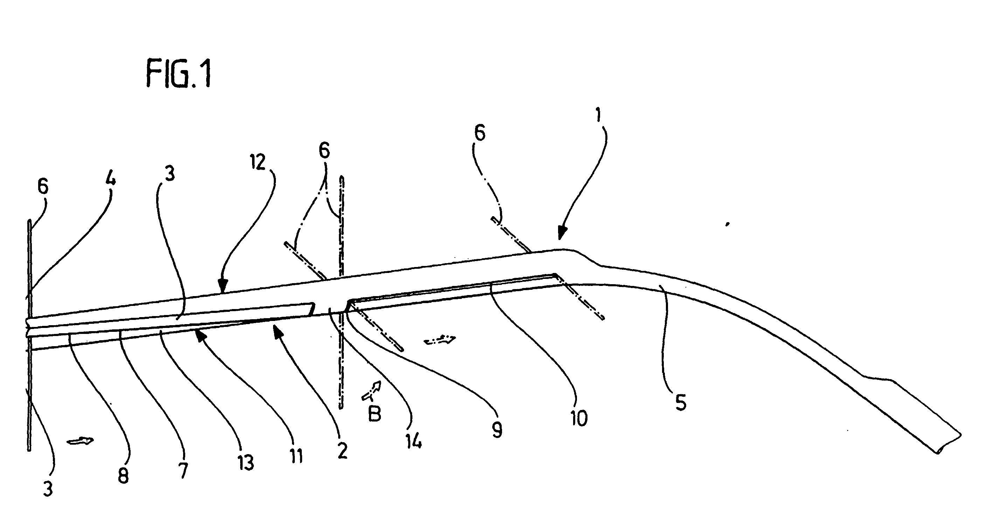

Bei dem in Figur 1 dargestellten Ausführungsbeispiel wird außerdem in einiger Entfernung von der Stirnseite 4 der Querschnitt des Drahts 1 über einen Biegebereich 5 geschwächt, beispielsweise auf die Hälfte des Querschnitts. In diesem Bereich läßt sich der Draht leichter biegen als in den übrigen Bereichen, und dieser Biegebereich 5 wird bei einem Rohrschaftinstrument mit einem gebogenem Rohr in dem Teil des Rohrs positioniert, der gebogen ist.

Nach dieser Bearbeitung des Außenumfangs des Drahts 1 werden diverse Einschnitte in den Draht eingebracht, und zwar mit Hilfe eines Werkzeugs, welches längs einer Linie eine Materialtrennung erzeugt. Es kann sich dabei insbesondere um eine Drahterosionsvorrichtung handeln, aber auch um Strahlvorrichtungen, beispielsweise Laserstrahlvorrichtungen oder Elektronenstrahlvorrichtungen. Im Ausführungsbeispiel der Figur 1 wird als Beispiel eine Drahterosionsvorrichtung dargestellt mit einem drahtförmigen Werkzeug 6, welches im Berührungsbereich mit dem zu bearbeitenden Draht 1 einen linienförmigen Trennschnitt im Material des Drahts 1 erzeugt.

Dieses Werkzeug 6 wird von der Stirnseite 4 des Drahts 1 her diametral in den Draht 1 eingeführt, und zwar derart, daß der dadurch entstehende diametrale vordere Einschnitt 7 an seiner Unterkante und an seiner Oberkante die Abflachungen 3 unter einem spitzen Winkel schneidet, so daß eine Schnittlinie 8 ausgebildet wird.

Der diametrale vordere Einschnitt 7 teilt den Draht 1 in zwei Hälften, und dieser Einschnitt 7 erstreckt sich über die Abflachungen 3 hinaus.

Am Ende des diametralen vorderen Einschnitts 7, der sich beispielsweise bei einem Draht 1 mit einem Durchmesser von 0,7 mm über eine Länge von 5,5 mm erstrecken kann, werden Werkzeug 6 und Draht 1 um die Längsachse des Drahts 1 relativ zueinander verdreht, und zwar um einen Winkel von 90°. Dies ist in Figur 1 durch den Pfeil B dargestellt. Dadurch werden am Ende des diametralen vorderen Einschnitts 7 zwei Quereinschnitte 9 im Draht 1 erzeugt, die sich über einen Winkel von 90° erstrecken und somit jeweils einen Quadranten des Drahtquerschnitts ausfüllen. Die Quereinschnitte 9 liegen sich diametral gegenüber, dazwischen verbleiben sich ebenfalls über einen Umfangswinkel von 90° erstreckende Bereiche ohne derartige Quereinschnitte.

Nach der Drehung des Drahts 1 relativ zum Werkzeug 6 wird das Werkzeug 6 weiter im Draht 1 geführt und erzeugt dort einen diametralen hinteren Einschnitt 10, der gegenüber dem diametralen vorderen Einschnitt 7 um 90° um die Drahtlängsachse gedreht ist und der sich beispielsweise über dieselbe Länge erstrecken kann wie der diametrale vordere Einschnitt 7. Der diametrale vordere Einschnitt 7, die beiden Quereinschnitte 9 und der diametrale hintere Einschnitt 10 teilen den Draht 1 in zwei Arme 11, 12, die am Ende des diametralen hinteren Einschnitts 10 einstückig mit dem verbleibenden Teil des Drahts 1 verbunden sind.

Diese beiden Arme 11, 12 bilden die Arme des scherenförmigen Werkzeugs 2. Um diese auch tatsächlich nach Art einer Schere einsetzen zu können, ist anschließend an das Einbringen der beschriebenen Einschnitte eine Verformung der Arme notwendig.

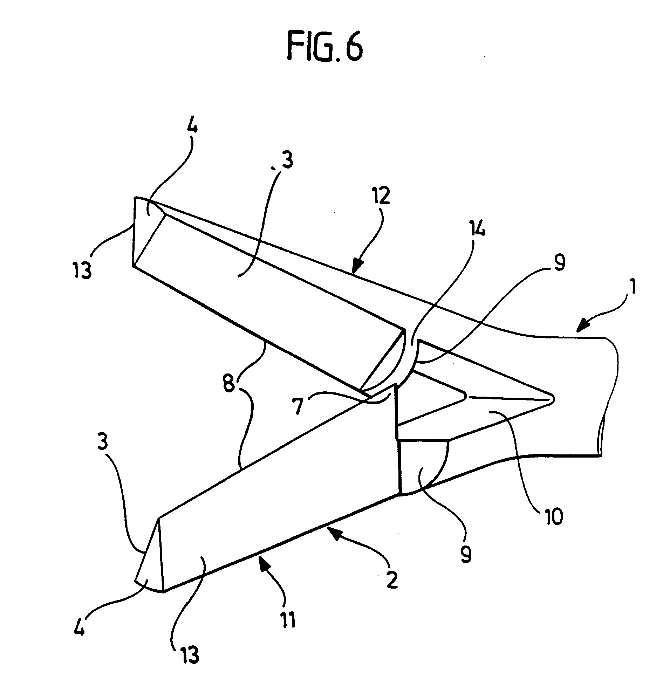

Zu diesem Zweck werden beide Arme zunächst in der Ebene des diametralen vorderen Einschnitts 7 spreizend auseinandergebogen, und zwar so weit, daß die Innenflächen 13 des diametralen vorderen Einschnitts 7 sich nicht mehr überlappen. Anschließend werden die beiden Arme in der Ebene des diametralen hinteren Einschnitts 10 gegeneinander verbogen, vorzugsweise so weit, bis die Außenflächen 14 der beiden Arme in einer Ebene liegen. In dieser Stellung, die in Figur 6 schematisch dargestellt ist, sind die Arme somit in zwei Ebenen elastisch aus ihrer ursprünglichen Ruhelage ausgelenkt. Durch eine an diesen Verformungsprozeß anschließende Wärmebehandlung wird diese Position, wie sie beispielsweise in Figur 6 dargestellt ist, fixiert. Dies erfolgt durch eine Gefügeumwandlung als Folge des Wärmebehandlungsprozesses.

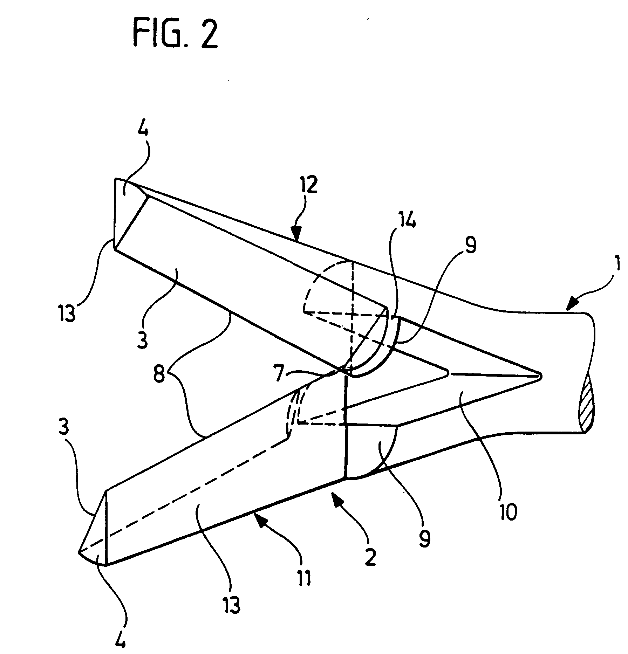

Damit ergibt sich eine neue Ruhestellung der beiden Arme, die etwa der Position in Figur 6 entspricht. Diese Ruheposition ist vorzugsweise so gewählt, daß die beiden Arme in der gespreizten Stellung sich noch ganz geringfügig überlappen. Wenn man nunmehr die beiden Arme durch elastisches weiteres Aufbiegen und elastisches Zurückbiegen des seitlich ausgeschwenkten Arms wieder aus der in Figur 6 dargestellten Wärmebehandlungsposition in eine Position bringt, bei der die beiden Arme im wesentlichen parallel verlaufen, legen sich die Innenflächen 13 des diametralen vorderen Einschnitts 7 in der Ruhestellung aneinander. Die Überlappung ist dabei minimal, wie dies in Figur 2 dargestellt ist. Außerdem werden die Innenflächen 13 elastisch gegeneinander gedrückt, so daß die beiden Arme 11 und 12 im Bereich ihrer Schnittlinien 8 unter einer bestimmten Vorspannung aneinander anliegen. Damit nimmt das scherenförmige Werkzeug die in Figur 2 dargestellte Ruhestellung ein, die gleichzeitig die Offenstellung des Werkzeugs bildet.

Zum Schließen des scherenförmigen Werkzeugs 2 wird über den Draht 1 eine Hülse geschoben, die relativ zum Draht 1 in axialer Richtung verschiebbar ist. Sie umgreift die beiden Arme 11, 12 insbesondere im Bereich des diametralen hinteren Einschnitts 10 und nähert diese beim weiteren Vorschieben der Hülse auf dem Draht 1 immer mehr aneinander an, wodurch die Arme 11, 12 elastisch in die angenäherte Schließstellung verschwenkt werden. Dabei gleiten die Innenflächen 13 der beiden Arme 11, 12 im Bereich der Schnittlinie 8 unter Vorspannung aneinander entlang, in diesem Bereich erhält man somit eine Schneidbewegung.

Nach der Ausführung eines Schnitts wird die auf dem Draht 1 verschiebbare Hülse wieder zurückgezogen, so daß sich die Arme 11, 12 wieder elastisch in ihre Ruhestellung begeben können, also in die geöffnete Stellung.

Die auf den Draht 1 aufgeschobene Hülse kann bei einem geradlinigen Rohrschaftinstrument ein Metallröhrchen sein, bei einem Rohrschaftinstrument mit gebogenem Rohr ist es günstig, wenn auf den Draht 1 als Hülse ein flexibler Schlauch aufgeschoben ist, beispielsweise ein Schlauch aus Polytetrafluorethylen. Dieser kann zwischen dem Draht 1 einerseits und dem umgebenden Rohr andererseits in axialer Richtung verschoben werden und dadurch die Öffnungs- und Schließbewegung des scherenförmigen Werkzeugs 2 steuern.

Bei dem Ausführungsbeispiel der Figuren 1 bis 3 und 6 sind die Arme 11, 12 in ihrer ursprünglichen Form belassen worden, das heißt sie sind geradlinig ausgebildet.

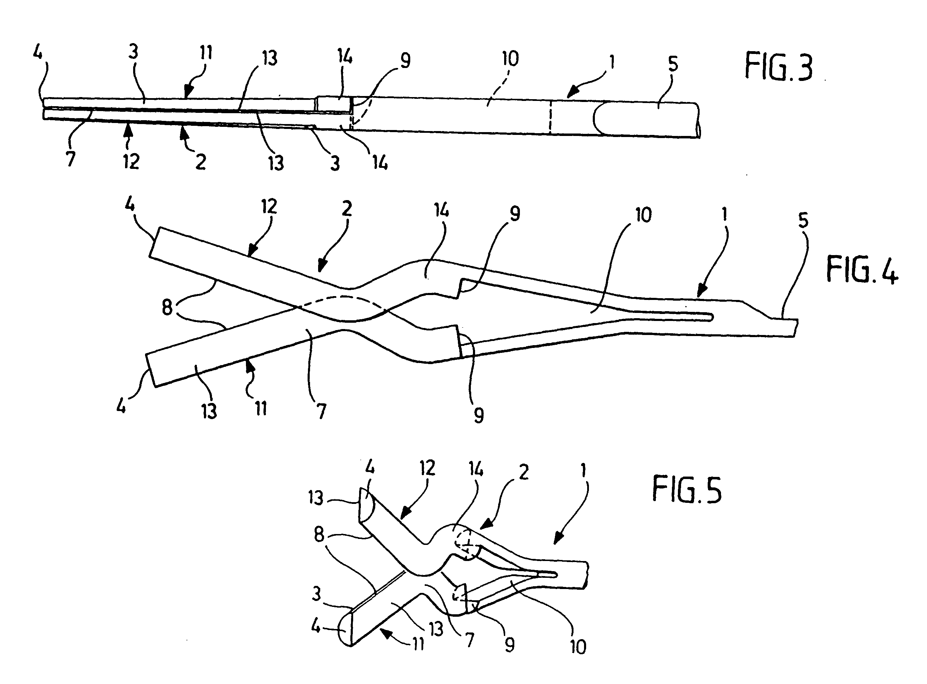

Bei dem Ausführungsbeispiel der Figuren 4 und 5 werden die Arme zusätzlich noch in der Ebene des diametralen vorderen Einschnitts 7 nach innen abgekröpft, so daß sich die Innenflächen 13 nicht im Übergangsbereich zum diametralen hinteren Einschnitt 10 überdecken, sondern an einer der Stirnseite 4 näheren Stelle. Es ist dadurch möglich, den Öffnungswinkel der beiden Arme größer auszugestalten, dies kann für bestimmte Anwendungsbereiche von Vorteil sein. Auch diese abkröpfende Verformung wird durch die beschriebene Wärmebehandlung stabilisiert.

## Patentansprüche

1. Scherenförmiges Werkzeug für ein chirurgisches Instrument mit zwei miteinander verbundenen Armen (11,12), die durch mehr oder weniger weites außenseitiges Übergreifen einer relativ zu dem scherenförmigen Werkzeug axial verschiebbaren, dieses umgreifenden Hülse elastisch zwischen einer gespreizten Offenstellung und einer angenäherten Schließstellung verschwenkbar sind, dadurch gekennzeichnet, daß zur Ausbildung der Arme (11, 12) ein Stab (1) aus elastischem Material von seinem freien Ende (4) her einen diametralen vorderen Einschnitt (7) aufweist, an den sich ein weiterer diametraler hinterer Einschnitt (10) anschließt, der gegenüber dem diametralen vorderen Einschnitt (7) um 90° um die Stablängsachse gedreht ist, daß die beiden diametralen Einschnitte (7, 10) durch quer zur Stablängsachse verlaufende Quereinschnitte (9) miteinander verbunden sind, die sich jeweils über gegenüberliegende Quadranten des Querschnitts zwischen den beiden diametralen Einschnitten (7, 10) erstrecken, während die beiden dazwischenliegenden Quadranten keine derartigen Quereinschnitte aufweisen, und daß die beiden Arme (11, 12) in der Ebene des diametralen vorderen Einschnitts (7) dauerhaft in die gespreizte Offenstellung aufgebogen sind.

2. Scherenförmiges Werkzeug nach Anspruch 1, dadurch gekennzeichnet, daß die beiden Arme (11, 12) zusätzlich in der Ebene des diametralen hinteren Einschnitts (10) dauerhaft einander überlappend gegeneinander gebogen sind.

3. Scherenförmiges Werkzeug nach Anspruch 1 oder 2, dadurch gekennzeichnet, daß der Stab (1) einen kreisförmigen Querschnitt aufweist.

4. Scherenförmiges Werkzeug nach einem der voranstehenden Ansprüche, dadurch gekennzeichnet, daß der Stab (1) zwei seitliche diametral gegenüberliegende, auBenseitige Abflachungen (3) aufweist, die mit dem diametralen vorderen Einschnitt (7) einen spitzen Winkel einschließen und zusammen mit der Innenfläche (13) des diametralen vorderen Einschnitts (7) eine Schneide (8) ausbilden.

5. Scherenförmiges Werkzeug nach einem der voranstehenden Ansprüche, dadurch gekennzeichnet, daß der Stab (1) aus einer Legierung besteht, die pseudoelastisches Verhalten zeigt.

6. Scherenförmiges Werkzeug nach einem der voranstehenden Ansprüche, dadurch gekennzeichnet, daß der Stab (1) durch die Schub- und Zugstange eines Rohrschaftinstruments gebildet wird, die in einem Rohr relativ zu diesem in axialer Richtung verschiebbar ist.

7. Scherenförmiges Werkzeug nach Anspruch 6, dadurch gekennzeichnet, daß der Stab (1) anschließend an das scherenförmige Werkzeug (2) einen Biegebereich (5) mit reduziertem Querschnitt aufweist.

8. Scherenförmiges Werkzeug nach Anspruch 7, dadurch gekennzeichnet, daß auf dem Stab (1) und innerhalb des Rohrs als das scherenförmige Werkzeug (2) umgreifende Hülse ein flexibler Schlauch angeordnet ist.

9. Scherenförmiges Werkzeug nach einem der voranstehenden Ansprüche, dadurch gekennzeichnet, daß die Arme (11, 12) in der Ebene des diametralen vorderen Einschnitts (7) nach innen abgekröpft sind.

10. Verfahren zur Herstellung eines scherenförmige Werkzeugs nach den Ansprüchen 1 bis 9, gekennzeichnet durch folgende Schritte:
a. man bringt durch ein linienförmig trennendes Werkzeug von der Stirnseite eines Stabs aus elastischem Material einen diametralen vorderen Einschnitt in den Stab ein und trennt diesen im Einschnittsbereich dadurch in zwei Hälften,
b. am Ende des diametralen vorderen Einschnitts dreht man den Stab relativ zum trennenden Werkzeug um die Stablängsachse um 90° und erzeugt dadurch zwei sich über 90° erstreckende Quereinschnitte,
c. man bringt durch das linienförmig trennende Werkzeug einen diametralen hinteren Einschnitt in den Stab ein, der um 90° gegenüber dem diametralen vorderen Einschnitt gedreht ist und sich von den Quereinschnitten weg nach hinten im Stab erstreckt,
d. man biegt die durch die Einschnitte entstandenen Arme in der Ebene des diametralen vorderen Einschnitts dauerhaft in eine gespreizte Offenstellung auf.

11. Verfahren nach Anspruch 10, dadurch gekennzeichnet, daß man die beiden Arme zusätzlich in der Ebene des diametralen hinteren Einschnitts dauerhaft aufeinanderzu biegt und sie dadurch in eine gegeneinander vorgespannte Position bringt.

12. Verfahren nach Anspruch 10 oder 11, dadurch gekennzeichnet, daß man zur dauerhaften Fixierung der Verbiegung der Arme in die Offenstellung und in eine gegeneinander vorgespannte Position den Stab einer Wärmebehandlung unterzieht.

13. Verfahren nach einem der Ansprüche 10 bis 12, dadurch gekennzeichnet, daß man als trennendes Werkzeug eine Drahterosionseinrichtung verwendet.

## Claims

1. A scissors-type implement for a surgical instrument, comprising two interconnected arms (11, 12) resiliently pivotable between an open position, in which they are spread apart, and a closed position, in which they are brought together, by means of a sleeve which surrounds the scissors-type implement and is axially displaceable relative thereto and engages over the outside of the said implement to a greater or lesser extent, characterised in that, to form the arms (11, 12), a rod (1) of resilient material has a diametral, front incision (7) starting from its free end (4), followed by a further diametral, rear incision (10) which, relative to the diametral front incision (7), is rotated through 90° about the longitudinal axis of the rod, in that the two diametral incisions (7, 10) are interconnected by transverse incisions (9) extending transversely to the longitudinal axis of the rod between the two diametral incisions (7, 10) over opposing quadrants of the cross-section, whereas the two quadrants arranged therebetween do not have such transverse incisions, and in that the two arms (11, 12) are permanently bent into the open position, in which they are spread apart, in the plane of the diametral front incision (7).

2. A scissors-type implement according to claim 1, characterised in that the two arms (11, 12) are additionally permanently bent towards one another so as to overlap in the plane of the diametral rear incision (10).

3. A scissors-type implement according to claim 1 or 2, characterised in that the rod (1) has a circular cross-section.

4. A scissors-type implement according to any one of the preceding claims, characterised in that the rod (1) has two lateral, diametrically opposed, outer flattened portions (3) which form an acute angle with the diametral front incision (7) and form a cutting edge (8) together with the inner surface (13) of the diametral front incision (7).

5. A scissors-type implement according to any one of the preceding claims, characterised in that the rod (1) is made of an alloy exhibiting pseudoelastic behaviour.

6. A scissors-type implement according to any one of the preceding claims, characterised in that the rod (1) is formed by the push-pull rod of a tubular-shaft instrument, the push-pull rod being axially displaceable in a tube relative to the latter.

7. A scissors-type implement according to claim 6, characterised in that the rod (1) has a bending region (5) of reduced cross-section adjacent to the scissors-type implement (2).

8. A scissors-type implement according to claim 7, characterised in that a flexible hose is arranged on the rod (1) and inside the tube to form a sleeve surrounding the scissors-type implement (2).

9. A scissors-type implement according to any one of the preceding claims, characterised in that the arms (11, 12) are bent inwards in the plane of the diametral front incision (7).

10. A method of manufacturing a scissors-type implement according to claims 1 to 9, characterised by the following steps:
a. a diametral front incision is made in a rod of resilient material by a linear cutting tool starting from the end of the rod, which is thus cut into two halves in the incision region;
b. at the end of the diametral front incision, the rod is rotated through 90° relative to the cutting tool about the longitudinal axis of the rod and two transverse incisions extending over 90° are thus formed;
c. a diametral rear incision is made in the rod by the linear cutting tool and is rotated through 90° relative to the diametral front incision and extends rearwards in the rod away from the transverse incisions;
d. the arms formed by the incisions are permanently bent into an open position, in which they are spread apart, in the plane of the diametral front incision.

11. A method according to claim 10, characterised in that the two arms are additionally permanently bent towards one another in the plane of the diametral rear incision and are thus brought into a mutually prestressed position.

12. A method according to claim 10 or 11, characterised in that, to fix the bent arms permanently in the open position and in a mutually prestressed position, the rod is subjected to heat treatment.

13. A method according to any one of claims 10 to 12, characterised in that a wire spark erosion device is used as a cutting tool.

## Revendications

1. Outil en forme de ciseaux pour un instrument chirurgical comportant deux bras (11, 12) reliés l'un à l'autre, qui sont susceptibles d'être pivotés de manière élastique entre une position ouverte écartée et une position fermée rapprochée, en les saisissant plus ou moins loin sur leur face extérieure au moyen d'une douille déplaçable axialement par rapport à l'outil en forme de ciseaux et entourant celui-ci, caractérisé en ce que pour former les bras (11, 12), une barre (1) en matériau élastique présente depuis son extrémité libre (4) une encoche (7) diamétrale antérieure, à laquelle se raccorde une autre encoche (10) diamétrale postérieure qui est tournée de 90° autour de l'axe longitudinal de la barre par rapport à l'encoche (7) diamétrale antérieure, en ce que les deux encoches (7, 10) diamétrales sont reliées l'une à l'autre par des encoches transversales (9) s'étendant transversalement à l'axe longitudinal de la barre, encoches qui s'étendent chacune sur des quarts de cercle opposés de la section entre les deux encoches (7, 10) diamétrales, tandis que les deux quarts de cercle situés entre eux ne présentent pas de telles encoches transversales, et en ce que les deux bras (11, 12) sont dépliés de manière durable dans la position ouverte écartée, dans le plan de l'encoche (7) diamétrale antérieure.

2. Outil en forme de ciseaux selon la revendication 1, caractérisé en ce que les deux bras (11, 12) sont pliés additionnellement l'un contre l'autre dans le plan de l'encoche (10) diamétrale postérieure de façon à se chevaucher mutuellement de manière durable.

3. Outil en forme de ciseaux selon l'une ou l'autre des revendications 1 et 2, caractérisé en ce que la barre (1) présente une section circulaire.

4. Outil en forme de ciseaux selon l'une quelconque des revendications précédentes, caractérisé en ce que la barre (1) présente deux méplats extérieurs (3) latéraux diamétralement opposés qui forment un angle aigu avec l'encoche (7) diamétrale antérieure et qui, conjointement avec la surface intérieure (13) de l'encoche (7) diamétrale antérieure, constituent un tranchant (8).

5. Outil en forme de ciseaux selon l'une quelconque des revendications précédentes, caractérisé en ce que la barre (1) est en un alliage qui présente un comportement pseudo-élastique.

6. Outil en forme de ciseaux selon l'une quelconque des revendications précédentes, caractérisé en ce que la barre (1) est formée par la tige de poussée et de traction d'un instrument à fût tubulaire, laquelle est déplaçable dans un tube en direction axiale par rapport à celui-ci.

7. Outil en forme de ciseaux selon la revendication 6, caractérisé en ce que la barre (1) présente en raccordement à l'outil (2) en forme de ciseaux une région de flexion (5) de section réduite.

8. Outil en forme de ciseaux selon la revendication 7, caractérisé en ce que sur la barre (1) et à l'intérieur du tube est agencé un tuyau flexible servant de douille entourant l'outil (2) en forme de ciseaux.

9. Outil en forme de ciseaux selon l'une quelconque des revendications précédentes, caractérisé en ce que les bras (11, 12) sont coudés vers l'intérieur dans le plan de l'encoche (7) diamétrale antérieure.

10. Procédé de fabrication d'un outil en forme de ciseaux selon les revendications 1 à 9, caractérisé par les étapes suivantes :
a. on ménage, au moyen d'un outil tranchant en forme de ligne depuis la face frontale d'une barre en matériau élastique, une encoche diamétrale antérieure dans la barre et on sépare ainsi celle-ci en deux moitiés dans la région de l'encoche,
b. à l'extrémité de l'encoche diamétrale antérieure, on tourne la barre de 90° autour de l'axe longitudinal de la barre, par rapport à l'outil tranchant, et on forme ainsi deux encoches transversales s'étendant sur 90°,
c. on ménage au moyen de l'outil tranchant en forme de ligne une encoche diamétrale postérieure dans la barre qui est tournée de 90° par rapport à l'encoche diamétrale antérieure et qui s'étend vers l'arrière dans la barre, en éloignement des encoches transversales,
d. on déplie de manière durable les bras produits par les encoches dans le plan de l'encoche diamétrale antérieure dans une position ouverte écartée.

11. Procédé selon la revendication 10, caractérisé en ce qu'on plie additionnellement et de manière durable les deux bras l'un vers l'autre dans le plan de l'encoche diamétrale postérieure et en ce qu'on les amène ainsi dans une position précontrainte l'un par rapport à l'autre.

12. Procédé selon l'une ou l'autre des revendications 10 et 11, caractérisé en ce que pour fixer de manière durable le coudage des bras dans la position ouverte et dans une position de précontrainte l'un par rapport à l'autre, on soumet la barre à un traitement thermique.

13. Procédé selon l'une quelconque des revendications 10 à 12, caractérisé en ce qu'on utilise comme outil tranchant un dispositif d'étincelage par fil.
